# EUROPEAN PATENT APPLICATION

(11) **EP 4 847 054 A1**
(43) Date of publication of application: **02.12.2026**
(21) Application number: 25777940.5
(22) Date of filing: 27.02.2025
(51) Int. Cl.: A61M 1/06

(54) **CONTROL METHOD AND APPARATUS FOR BREAST PUMP, BREAST PUMP, AND STORAGE MEDIUM**

(30) Priority: 29.03.2024 CN 202410379823
(71) Applicant: Shenzhen Lute Jiacheng Supply Chain Management Co., Ltd., Shenzhen, Guangdong 518000 (CN)
(72) Inventor: WANG, Dandan, Shenzhen, Guangdong 518000 (CN)
(74) Representative: Winter, Brandl - Partnerschaft mbB
(86) International application number: PCT/CN2025/079529
(87) International publication number: WO 2025/200920

(57) **Abstract**

Disclosed in the present application is a control method for a breast pump. The control method comprises: measuring the temperature of a negative pressure generator of a breast pump; determining whether the temperature of the negative pressure generator is greater than a preset temperature threshold value; after it is determined that the temperature of the negative pressure generator is greater than the preset temperature threshold value, controlling the negative pressure generator to operate in a first operating mode; and after it is determined that the temperature of the negative pressure generator is less than or equal to the preset temperature threshold value, controlling the negative pressure generator to operate in a second operating mode, wherein the output power of the negative pressure generator in the second operating mode is greater than the output power of the negative pressure generator in the first operating mode. Therefore, the problem of heat generation of a negative pressure generator of a breast pump can be ameliorated, and thus the problem of suction attenuation caused by the heat generation of the negative pressure generator is ameliorated.

## Description

### RELATED APPLICATIONS

This application claims priority to Chinese Patent Application No. 202410379823.3 filed on March 29, 2024, and the entire contents of which are incorporated herein by reference.

### TECHNICAL FIELD

Embodiments of the present disclosure relate to the technical field of maternal and infant appliances, and in particular, to a control method and a control apparatus for a breast pump, a breast pump, and a storage medium.

### BACKGROUND

Breast pumps are auxiliary tools configured to suck out breast milk accumulated in mammary glands. Common breast pumps can be divided into two types, i.e., unilateral/single-sided electric breast pumps or bilateral/double-sided electric breast pumps. The breast pumps help unblock mammary glands and promptly empty breast milk. The beast pumps generally use built-in vacuum pumps and solenoid valves. For portability and convenience, rechargeable batteries are usually built in as well.

With technological advancements, the breast pumps have gradually evolved from split-type breast pumps to integrated-wearable-type breast pumps. The volumes of wearable breast pumps are also gradually developing in a trend toward miniaturization. Vacuum generators are important components of breast pumps, and are configured to provide negative pressure required for milk expression. Heat generation of the vacuum generators has been a challenge that people have sought to solve for a long time, as excessive heat can potentially lead to a shortened lifespan of the vacuum generators and a suction loss.

### SUMMARY

In view of the deficiencies in the related art, the present disclosure provides a control method and device for a breast pump, a breast pump, and a storage medium, which can improve the heating problem of the vacuum generator of the breast pump.

To achieve the above objective, one technical solution adopted in the present disclosure is to provide a control method for a breast pump, including:
detecting a temperature of a vacuum generator of the breast pump;
determining whether the temperature of the vacuum generator is higher than a preset temperature threshold;
controlling the vacuum generator to operate in a first operating mode in a case where the temperature of the vacuum generator is determined to be higher than the preset temperature threshold; and
controlling the vacuum generator to operate in a second operating mode in a case where the temperature of the vacuum generator is determined be lower than or equal to the preset temperature threshold; where an output power of the vacuum generator in the second operating mode is higher than the output power of the vacuum generator in the first operating mode.

In some embodiments, the controlling the vacuum generator to operate in the first operating mode includes: in one operating cycle of the first operating mode, controlling the vacuum generator to operate at a first suction value in a first time period and controlling the vacuum generator to operate at a second suction value lower than the first suction value in a second time period;
where the controlling the vacuum generator to operate in the second operating mode includes: in one operating cycle of the second operating mode, controlling the vacuum generator to operate at the first suction value.

In some embodiments, the controlling the vacuum generator to operate in the first operating mode includes: in one operating cycle of the first operating mode, controlling the vacuum generator to operate at a first suction value in a first time period and controlling the vacuum generator to stop operating in a second time period;
where the controlling the vacuum generator to operate in the second operating mode includes: in one operating cycle of the second operating mode, controlling the vacuum generator to operate at the first suction value.

In some embodiments, the controlling the vacuum generator to operate in the first operating mode in a case where the temperature of the vacuum generator is determined to be higher than the preset temperature threshold includes:
in a case where the temperature of the vacuum generator is determined to be higher than the preset temperature threshold, further determining whether a user of the breast pump is currently in a milk letdown phase based on a milk-pumping flow rate detected in real time by the breast pump; and
in a case where the user is determined to be currently in the milk letdown phase, entering the first operating mode only after the current milk letdown phase is determined to end based on the milk-pumping flow rate.

In some embodiments, the determining whether the user of the breast pump is currently in the milk letdown phase based on the milk-pumping flow rate detected in real time by the breast pump includes:
determining whether the milk-pumping flow rate detected in real time is higher than a preset flow rate threshold; and
determining that the user is currently in the milk letdown phase in a case where the milk-pumping flow rate is higher than the preset flow rate threshold.

In some embodiments, the controlling the vacuum generator to operate in the first operating mode in a case where the temperature of the vacuum generator is determined to be higher than the preset temperature threshold includes:
controlling a massage unit of the breast pump to work in the second time period, in a case where the temperature of the vacuum generator is determined to be higher than the preset temperature threshold.

In some embodiments, the control method further includes:
setting a duration ratio of the first time period and a duration ratio of the second time period relative to the operating cycle according to an amplitude of the first suction value; where the greater the first suction value is, the greater the duration ratio of the second time period to the operating cycle is set to be.

To achieve the above objective, another technical solution adopted in the present disclosure is to provide a breast pump, which includes a processor and a memory electrically connected to the processor, the memory stores a computer program, and the processor may be configured to call the computer program to execute the above control method for the breast pump.

To achieve the above objective, a further technical solution adopted in the present disclosure is to provide a storage medium, which is configured to store a computer program, and the computer program can be executed to implement the above control method for the breast pump.

To achieve the above objective, another further technical solution adopted in the present disclosure is to provide a control apparatus for a breast pump, including:
a detection module, configured to detect a temperature of a vacuum generator of the breast pump;
a determination module, configured to determine whether the temperature of the vacuum generator is higher than a preset temperature threshold; and
an execution module, configured to control the vacuum generator to operate in a first operating mode in a case where the temperature of the vacuum generator is determined to be higher than the preset temperature threshold, and control the vacuum generator to operate in a second operating mode in a case where the temperature of the vacuum generator is determined be lower than or equal to the preset temperature threshold; where an output power of the vacuum generator in the second operating mode is higher than the output power of the vacuum generator in the first operating mode.

To achieve the above objective, yet another technical solution adopted in the present disclosure is to provide a breast pump, which includes a processor, and a temperature sensor and a vacuum generator both electrically connected to the processor;
where the temperature sensor is configured to detect a temperature of the vacuum generator of the breast pump;
the processor is configured to determine whether the temperature of the vacuum generator is higher than a preset temperature threshold, control the vacuum generator to operate in a first operating mode in a case where the temperature of the vacuum generator is determined to be higher than the preset temperature threshold, and control the vacuum generator to operate in a second operating mode in a case where the temperature of the vacuum generator is determined be lower than or equal to the preset temperature threshold; where an output power of the vacuum generator in the second operating mode is higher than the output power of the vacuum generator in the first operating mode.

Embodiments of the present disclosure provides a control method for a breast pump, including: detecting a temperature of a vacuum generator of the breast pump; determining whether the temperature of the vacuum generator is higher than a preset temperature threshold; controlling the vacuum generator to operate in a first operating mode in a case where the temperature of the vacuum generator is determined to be higher than the preset temperature threshold; and controlling the vacuum generator to operate in a second operating mode in a case where the temperature of the vacuum generator is determined to be lower than or equal to the preset temperature threshold, where an output power of the vacuum generator in the second operating mode is higher than that in the first operating mode. This may mitigate the heating problem of the vacuum generator of the breast pump, and thus mitigate the suction attenuation problem of the vacuum generator caused by heating.

### BRIEF DESCRIPTION OF THE DRAWINGS

In order to explain the technical solutions in some embodiments of the present disclosure or the related art more clearly, the following will briefly introduce the drawings required for describing some embodiments of the present disclosure or the related art. Obviously, the drawings in the following description are only some embodiments of the present disclosure. For those skilled in the art, other drawings may be obtained based on the structures shown in these drawings without creative effort.
FIG. 1 is a schematic flowchart of a control method for a breast pump according to a first embodiment of the present disclosure.
FIG. 2 is a schematic flowchart of a control method for a breast pump according to a second embodiment of the present disclosure.
FIG. 3 is a schematic flowchart of the operation of determining whether a user is currently in a milk letdown phase based on a milk-pumping flow rate detected in real time by the breast pump according to a third embodiment of the present disclosure.
FIG. 4 is a schematic block diagram of a control apparatus for a breast pump according to some embodiments of the present disclosure.
FIG. 5 is a schematic diagram of a hardware structure of a breast pump according to some embodiment of the present disclosure.
FIG. 6 is a schematic diagram of another hardware structure of a breast pump according to some embodiments of the present disclosure.

The realization of the objectives, functional features, and advantages of the present disclosure will be further described in conjunction with the embodiments and with reference to the accompanying drawings.

### DETAILED DESCRIPTION

In the present disclosure, the terms "arranged with", "provided with", and "connected" should be understood broadly. For example, the connection may be a fixed connection, a detachable connection, or an integral structure; it may be a mechanical connection or an electrical connection; it may be a direct connection, an indirect connection through an intermediary, or internal communication between two devices, components, or constituent parts. For those skilled in the art, the specific meanings of the above terms in the present disclosure may be understood according to specific circumstances.

The orientation or positional relationships indicated by terms such as "center", "longitudinal", "transverse", "length", "width", "thickness", "upper", "lower", "front", "rear", "left", "right", "vertical", "horizontal", "top", "bottom", "inner", "outer", "radial", "circumferential", etc. may be based on the orientation or positional relationships shown in the drawings, and may be only for the convenience of describing the present disclosure and simplifying the description, rather than indicating or implying that the device or element referred to must have a specific orientation, be constructed and operate in a specific orientation, and therefore cannot be construed as limitations to the present disclosure.

Furthermore, the terms "first" and "second" may be for descriptive purposes only and may be not to be construed as indicating or implying relative importance or implicitly indicating the number of technical features indicated. Thus, features defined as "first" or "second" may explicitly or implicitly include at least one such feature. In the description of the present disclosure, "a plurality" means at least two, such as two, three, etc., unless otherwise specifically defined.

Moreover, in addition to being intended to indicate orientation or positional relationships, the aforementioned terms may further be configured to indicate other meanings. For example, the term "upper" may in some cases be configured to indicate a certain attachment or connection relationship. For those skilled in the art, the specific meanings of these terms in the present disclosure may be understood according to specific circumstances.

To make the objectives, technical solutions, and advantages of the present disclosure clearer, the present disclosure will be further described in detail below with reference to the accompanying drawings and embodiments. It should be understood that the specific embodiments described herein may be only intended to explain the present disclosure and may be not to limit the present disclosure.

A vacuum generator in some embodiments of the present disclosure may be, for example, an electric piston pump, an electric vortex pump, an electric diaphragm pump, an air-driven vacuum pump, a piezoelectric pump, or the like, which may be not limited in some embodiments of the present disclosure. These vacuum generators exhibit varying degrees of heat generation. A piezoelectric pump may be a type of fluid actuator. It does not need an additional drive motor. Instead, it utilizes the inverse piezoelectric effect of piezoelectric material to cause the piezoelectric vibrator to deform, and then uses the deformation to change the volume of a pump chamber to achieve fluid output, or uses the piezoelectric vibrator to generate waves to drive the fluid and create negative pressure. Piezoelectric pumps may be small in size and have low noise. However, there may be also some problems. For example, the piezoelectric pumps may have much more serious heat generation compared to conventional air pumps. In some embodiments, the piezoelectric pump may be a piezoelectric ceramic pump.

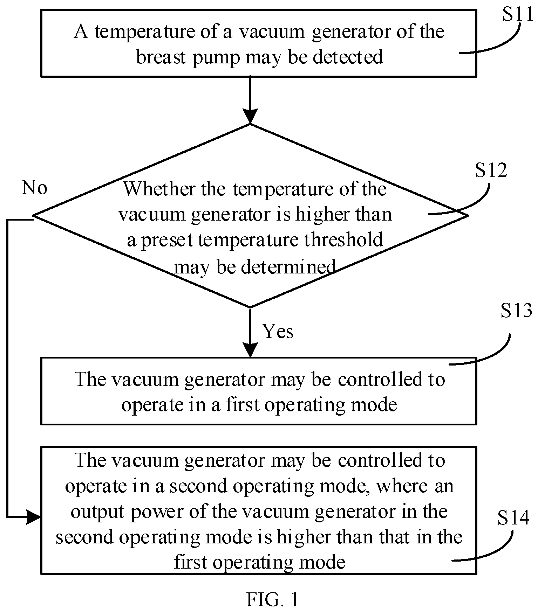

As shown in FIG. 1, FIG. 1 is a schematic flowchart of a control method for a breast pump according to a first embodiment of the present disclosure.

In some embodiments, the control method for the breast pump may include the following operations.

In an operation S11, a temperature of a vacuum generator of the breast pump may be detected.

A temperature sensor may be arranged on the vacuum generator or at a position around and adjacent to the vacuum generator (also known as "immediate vicinity of the vacuum generator") to detect the temperature of the vacuum generator. For example, the temperature sensor may be arranged on a pump housing of the vacuum generator or at a position around the pump housing.

In an operation S12, whether the temperature of the vacuum generator is higher than a preset temperature threshold may be determined.

Based on the determination in the operation S12 that the temperature of the vacuum generator is higher than the preset temperature threshold, an operation S13 may be executed. In the operation S13, the vacuum generator may be controlled to operate in a first operating mode.

Based on the determination in the operation S12 that the temperature of the vacuum generator is lower than or equal to the preset temperature threshold, an operation S14 may be executed. In the operation S14, the vacuum generator may be controlled to operate in a second operating mode, where an output power of the vacuum generator in the second operating mode is higher than that in the first operating mode.

The currently detected temperature of the vacuum generator may be compared with a safety temperature threshold preset in the system. In a case where the temperature of the vacuum generator is higher than the preset temperature threshold, it indicates that the vacuum generator might be overheated. To prevent damage to the component or reduce impact on operational safety and efficiency, the vacuum generator may be controlled to enter the first operating mode. This mode may involve measures such as reducing output power, operating intermittently, or activating a cooling mechanism, to reduce heat generation and ensure that the temperature may be maintained within a safe range. In a case where the temperature of the vacuum generator is lower than or equal to the preset temperature threshold, it indicates that the device may be operating in a normal temperature, and an operating efficiency can be safely improved. In this case, the vacuum generator may be controlled to enter the second operating mode. In the second operating mode, the vacuum generator operates at a higher output power, thereby providing a stronger suction force or a faster suction speed to meet the user's demand for efficient milk expression.

In some embodiments, the operation of controlling the vacuum generator to operate in the first operating mode may include the following operations: within one operating cycle of the first operating mode, controlling the vacuum generator to operate at a first suction value in a first time period; and controlling the vacuum generator to operate at a second suction value lower than the first suction value in a second time period. The operation of controlling the vacuum generator to operate in the second operating mode may include: within one operating cycle of the second operating mode, controlling the vacuum generator to operate at the first suction value. In the first operating mode, within each complete operating cycle, the system may first set a time period (the first time period), during which the vacuum generator may operate at the first suction value which is higher to ensure efficient extraction or delivery of the medium. Subsequently, in the second time period within the same operating cycle, the suction value of the vacuum generator may be reduced to the second suction value that is lower than the first suction value. This strategy may be intended to reduce heat accumulation caused during continuous high-intensity operation, prevent overheating of the vacuum generator, thereby prolonging the service life of the device and optimizing energy utilization efficiency. In the second operating mode: the vacuum generator always operates at the first suction value throughout a single operating cycle. This means that the device may be in a full-power operation state in this mode, making it suitable for scenarios requiring rapid or continuous high-efficiency operation. However, it may increase the heat generation and energy consumption of the vacuum generator in this case. In this way, the control system may flexibly switch the operating mode of the vacuum generator based on actual demands, temperature conditions, and energy consumption requirements, so as to achieve a balance between performance and heating control.

In some other embodiments, the operation of controlling the vacuum generator to operate in the first operating mode may include the following operations: within one operating cycle of the first operating mode, controlling the vacuum generator to operate at a first suction value in a first time period; and controlling the vacuum generator to stop operating in a second time period. The operation of controlling the vacuum generator to operate in the second operating mode may include: within one operating cycle of the second operating mode, controlling the vacuum generator to operate at the first suction value. In the first operating mode, within each complete operating cycle, a time period (the first time period) may be first set, during which the vacuum generator may operate normally at a set first suction value. Next, in the second time period within the same operating cycle, the vacuum generator may be stopped. The design of "intermittent operation" aims to reduce heat accumulation during continuous operation of the equipment by incorporating a shutdown/idle period, thereby reducing the occurrence of overheating and also saving energy consumption. In the second operating mode, the vacuum generator may keep operating at full speed during the entire operating cycle, that is, always operates at the first suction value. This ensures that the equipment can continuously provide the required suction capacity, making it suitable for scenarios that requires continuous and efficient operation; however, this may also cause the temperature of the vacuum generator to rise rapidly. Through the selection and switching of these two different operating modes, the system may intelligently regulate the operating state of the vacuum generator based on actual demands, heat dissipation conditions, and energy consumption requirements, so as to realize the balance and optimization between performance and energy saving, and between stability and safety.

In some embodiments, the operation of controlling the vacuum generator to operate in the first operating mode based on the determination that the temperature of the vacuum generator is higher than the preset temperature threshold may include the following operations: controlling a massage unit of the breast pump to operate in the second time period in a case where the temperature of the vacuum generator is determined to be higher than the preset temperature threshold. In some embodiments, the strategy for the temperature control of the vacuum generator may be further refined: in a case where the system detects that the operating temperature of the vacuum generator is greater than the preset temperature threshold, the system switches to the first operating mode to prevent overheating of the vacuum generator and protect the performance and safety of the equipment. The specific operations may be as follows: in the original first operating mode, based on the determination that the temperature is too high, in addition to maintaining the operation of the vacuum generator at the first suction value in the first time period, a new mechanism may be added: in the second time period, not only the operation of the vacuum generator may be reduced or suspended (as mentioned above), but also the massage unit function of the breast pump may be started or enhanced. Through such adjustment, the massage unit may serve to disperse the user's demand for suction intensity, indirectly reducing the working load of the vacuum generator, thereby helping it cool down. This strategy may not only ensure the user's comfort, but also ensure that the equipment will not fail due to long-term high-temperature operation, thereby prolonging the service life and safety of the equipment.

In some embodiments, the control method further may include: setting duration ratios of the first time period and the second time period relative to the operating cycle according to an amplitude of the first suction value, where the greater the first suction value is, the greater the duration ratio of the second time period relative to the operating cycle may be set to be. In the above control method for the vacuum generator, the intelligent setting of the duration ratios of different time periods in the operating cycle may be further included: in a case where the operating mode of the vacuum generator is determined according to the first suction value, the system dynamically adjusts the proportional distribution of the first time period and the second time period in the entire operating cycle according to the amplitude of the first suction value. The specific rule may be as follows: with the increase of the first suction value, that is, when the vacuum generator operates at a higher suction intensity, the system will appropriately increase the proportion of the second time period in the entire operating cycle to prevent overheating and prolong the service life of the equipment. That may be to say, within the same operating cycle, the time for the vacuum generator to work at high intensity (the first suction value) may be reduced, and the time for the vacuum generator to work at low power or stop (the second time period) may be increased. Such a design may ensure effective control of the operating temperature of the vacuum generator while ensuring the necessary suction effect, so as to realize the balance and optimization between performance and safety, efficiency, and durability.

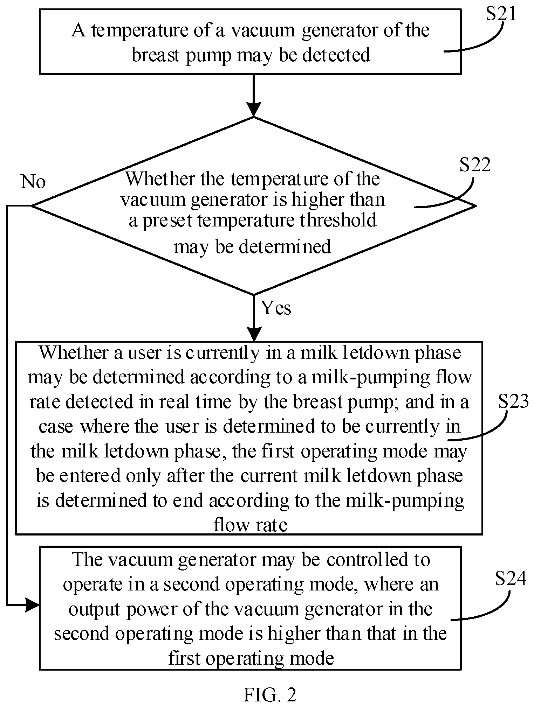

As shown in FIG. 2, FIG. 2 is a schematic flowchart of a control method for a breast pump according to a second embodiment of the present disclosure.

In some embodiments, the operation of controlling the vacuum generator to operate in the first operating mode based on the determination that the temperature of the vacuum generator is higher than the preset temperature threshold may include the following operations: based on the determination that the temperature of the vacuum generator is higher than the preset temperature threshold, further determining whether a user is currently in a milk letdown phase based on a milk-pumping flow rate detected in real time by the breast pump; and in a case where the user is determined to be currently in the milk letdown phase, entering the first operating mode only after the current milk letdown phase is determined to end based on the milk-pumping flow rate.

In some embodiments, the control method for the breast pump may specifically include the following operations.

In an operation S21: a temperature of a vacuum generator of the breast pump may be detected.

In an operation S22: whether the temperature of the vacuum generator is higher than a preset temperature threshold may be determined.

Based on the determination in the operation S22 that the temperature of the vacuum generator is higher than the preset temperature threshold, an operation S23 may be executed. In the operation S23, whether a user is currently in a milk letdown phase may be determined based on a milk-pumping flow rate detected in real time by the breast pump; and in a case where the user is determined to be currently in the milk letdown phase, the first operating mode may be entered only after the current milk letdown phase is determined to end based on the milk-pumping flow rate.

Based on the determination in the operation S22 that, the temperature of the vacuum generator is lower than or equal to the preset temperature threshold, an operation S14 may be executed. In the operation S14, the vacuum generator may be controlled to operate in a second operating mode, where an output power of the vacuum generator in the second operating mode is higher than that in the first operating mode.

In some embodiments, the temperature control strategy for the vacuum generator may be more finely adjusted. In a case where the operating temperature of the vacuum generator is detected to be higher than the preset temperature threshold, the system may first consider entering the first operating mode to reduce the working intensity and heat generation. On this basis, the determination of the milk letdown phase (a phase of rapid milk outflow), which is a physiological characteristic in the milk pumping process, may be further introduced. The specific operations may be as follows. 1. when the temperature of the vacuum generator is found to be over high, the system pauses to switch to the first operating mode immediately. 2. the system monitors the current milk-pumping flow rate in real time to determine whether the user is in the milk letdown phase; when the milk letdown phase is being experienced, that is, the user is in a state of a large amount of milk outflow, even if the vacuum generator is overheated, the current operating state may be temporarily maintained until the milk letdown phase ends naturally to ensure effective breast milk extraction and avoid waste or discomfort. 3. after the end of the milk letdown phase, the system adjusts the vacuum generator to the first operating mode, and reduces the operating temperature of the vacuum generator by reducing the suction force in the first time period or stopping the operation in the second time period. This intelligent adjustment method may not only ensure the effective milk pumping effect during the user's milk production peak, but also effectively prevent the performance degradation or damage of the vacuum generator caused by continuous high temperature.

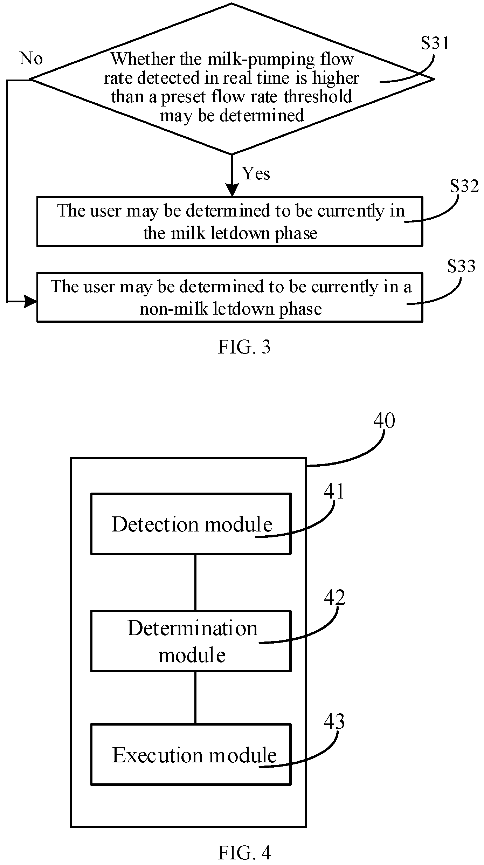

As shown in FIG. 3, FIG. 3 is a schematic flowchart of the operation of determining whether a user is currently in a milk letdown phase based on a milk-pumping flow rate detected in real time by the breast pump according to a third embodiment of the present disclosure.

In some embodiments, the operation of determining whether the user is currently in a milk letdown phase based on the milk-pumping flow rate detected in real time by the breast pump may specifically include the following operations.

In an operation S31: whether the milk-pumping flow rate detected in real time is higher than a preset flow rate threshold may be determined.

Based on the determination in the operation S31 that the milk-pumping flow rate is higher than the preset flow rate threshold, an operation S32 may be executed. In the operation S32, the user may be determined to be currently in the milk letdown phase.

Based on the determination in the operation S31 that the milk-pumping flow rate is lower than or equal to the preset flow rate threshold, an operation S33 may be executed. In the operation S33, the user may be determined to be currently in a non-milk letdown phase.

In this control method, the flow rate change during milk pumping may be monitored and determined in real time. In the operation S31, the system may continuously monitor the actual breast milk flow rate collected by the breast pump and compares the actual breast milk flow rate with the preset flow rate threshold. In a case where the detected milk-pumping flow rate is higher than the preset flow rate threshold, it may be indicated that the user is in the milk letdown phase, that is, a state of rapid milk outflow. In this case, the operation S32 is executed, where the system may determine the current state as the milk letdown phase, during which the operating efficiency of the vacuum generator may be maintained or appropriately improved to ensure efficient milk pumping, and the temperature change may be also closely monitored to prevent overheating. In a case where the detected milk-pumping flow rate is lower than or equal to the preset flow rate threshold, it may be considered that the breast pump is currently in the non-milk letdown phase, that is, the phase where the milk outflow speed may be relatively stable or slow. In this case, the operation S33 is executed, where the system may determine the current state as the non-milk letdown phase, and in this phase, the vacuum generator may be regulated according to the preset first operating mode (such as adjusting the suction force in different time periods or stopping the operation as mentioned above), which not only ensures an appropriate milk pumping effect, but also can effectively manage the heating of the equipment and prolong the service life.

As shown in FIG. 4, FIG. 4 is a schematic block diagram of a control apparatus for a breast pump according to some embodiments of the present disclosure.

In some embodiments, the control apparatus 40 for the breast pump may include a detection module 41, an acquisition module 42, and a setting module 43.

The detection module 41 may be configured to detect a temperature of a vacuum generator of the breast pump.

The determination module 42 may be configured to determine whether the temperature of the vacuum generator is higher than a preset temperature threshold.

The execution module 43 may be configured to control the vacuum generator to operate in a first operating mode in a case where the determination module 42 determines that the temperature of the vacuum generator is higher than the preset temperature threshold, and control the vacuum generator to operate in a second operating mode in a case where the determination module determines that the temperature of the vacuum generator is lower than or equal to the preset temperature threshold, where an output power of the vacuum generator in the second operating mode is higher than that in the first operating mode.

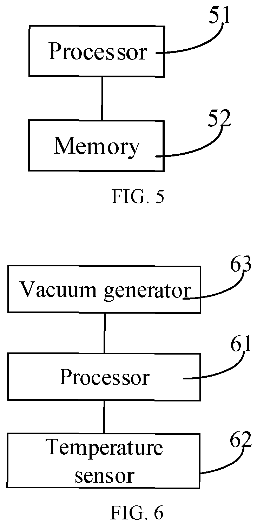

As shown in FIG. 5, FIG. 5 is a schematic diagram of a hardware structure of a breast pump according to some embodiment of the present disclosure.

In some embodiments, the breast pump 50 may include a processor 51 and a memory 52 electrically connected to the processor 51, the memory 52 stores a computer program, and the processor 51 may be configured to call the computer program to execute the control method for the breast pump according to any one of the above embodiments.

As shown in FIG. 6, FIG. 6 is a schematic diagram of another hardware structure of a breast pump according to some embodiments of the present disclosure.

In some embodiments, the breast pump may include a processor 61, and a temperature sensor 62 and a vacuum generator 63 both electrically connected to the processor 61. The temperature sensor 62 may be configured to detect a temperature of the vacuum generator 63 of the breast pump. The processor 61 may be configured to determine whether the temperature of the vacuum generator 63 is higher than a preset temperature threshold, control the vacuum generator 63 to operate in a first operating mode in a case where the temperature of the vacuum generator 63 is determined to be higher than the preset temperature threshold, and control the vacuum generator 63 to operate in a second operating mode in a case where the temperature of the vacuum generator 63 is determined to be lower than or equal to the preset temperature threshold, where an output power of the vacuum generator 63 in the second operating mode is higher than that in the first operating mode.

In some embodiments, the processor 61 may be configured to control the vacuum generator 63 to operate in the first operating mode specifically in that: within one operating cycle of the first operating mode, the processor 61 may be configured to control the vacuum generator 63 to operate at a first suction value in a first time period and control the vacuum generator 63 to operate at a second suction value lower than the first suction value in a second time period. The processor 61 may be configured to control the vacuum generator 63 to operate in the second operating mode specifically in that: within one operating cycle of the second operating mode, the processor 61 may be configured to control the vacuum generator 63 to operate at the first suction value.

In some other embodiments, the processor 61 may be configured to control the vacuum generator 63 to operate in the first operating mode specifically in that: within one operating cycle of the first operating mode, the processor 61may be configured to control the vacuum generator 63 to operate at a first suction value in a first time period and controls the vacuum generator 63 to stop operating in a second time period. The processor 61 may be configured to control the vacuum generator 63 to operate in the second operating mode specifically in that: within one operating cycle of the second operating mode, the processor 61 may be configured to control the vacuum generator to operate at the first suction value.

In some embodiments, the breast pump may further include a flow monitoring unit configured to detect a milk-pumping flow rate of the breast pump. The processor 61 may be configured to control the vacuum generator 63 to operate in the first operating mode in a case where the temperature of the vacuum generator 63 is determined to be higher than the preset temperature threshold specifically in that: in a case where the temperature of the vacuum generator 63 is determined to be higher than the preset temperature threshold, the processor 61 may be further configured to determine whether a user is currently in a milk letdown phase based on the milk-pumping flow rate detected in real time by the breast pump; and in a case where the user is determined to be currently in the milk letdown phase, the processor 61 may be configured to enter the first operating mode only after the current milk letdown phase is determined to end based on the milk-pumping flow rate.

In some embodiments, the processor 61 may be configured to determine whether the user is currently in the milk letdown phase based on the milk-pumping flow rate detected in real time by the breast pump specifically in that: the processor 61 may be configured to obtain a real-time milk-pumping flow rate value detected by the flow monitoring unit and determine whether the milk-pumping flow rate detected in real time is higher than the preset flow rate threshold; the processor 61 may be configured to determine that the user is currently in the milk letdown phase in a case where the milk-pumping flow rate is higher than the preset flow rate threshold.

In some embodiments, the breast pump may further include a massage unit. The processor 61 may be configured to control the vacuum generator 63 to operate in the first operating mode in a case where the temperature of the vacuum generator 63 is determined to be higher than the preset temperature threshold specifically in that: the processor 61 may be configured to control the massage unit of the breast pump to work in the second time period after in a case where the temperature of the vacuum generator 63 is determined to be higher than the preset temperature threshold.

Embodiments of the present disclosure may further provide a storage medium, which stores a computer program that, when executed by a processor, can implement the control method for the breast pump according to any one of the above embodiments.

The computer program may be stored in the above storage medium in the form of a software product, including several instructions to enable a device or a processor to execute all or part of the operations of the methods in the various embodiments of the present disclosure.

A storage medium may be a medium for storing a certain discontinuous physical quantity in a computer memory. The aforementioned storage medium may be: a USB flash drive, a mobile hard disk, a Read-Only Memory (ROM), a Random Access Memory (RAM), a magnetic disk, an optical disk, or various other media that can store program codes.

In the several embodiments provided in the present disclosure, it should be understood that the disclosed method and device may be implemented in other ways. For example, the device embodiments described above may be only schematic; for example, the division of modules or units may be only a logical function division, and there may be other division modes in actual implementation, for example, multiple units or components may be combined or integrated into another system, or some features may be ignored or not executed.

Units described as separate components may or may not be physically separate, and components shown as units may or may not be physical units, that is, they may be located in one place, or may be distributed on a plurality of network units. Some or all of the units may be selected according to actual needs to achieve the objectives of the solutions of the embodiments.

In addition, each functional unit in each embodiment of the present disclosure may be integrated into one processing unit, or each unit may exist alone physically, or two or more units may be integrated into one unit. The above integrated units may be implemented in the form of hardware, or in the form of software functional units.

Embodiments of the present disclosure provides a control method for a breast pump, including: detecting a temperature of a vacuum generator of the breast pump; determining whether the temperature of the vacuum generator is higher than a preset temperature threshold; controlling the vacuum generator to operate in a first operating mode in a case where the temperature of the vacuum generator is determined to be higher than the preset temperature threshold; and controlling the vacuum generator to operate in a second operating mode in a case where the temperature of the vacuum generator is determined to be lower than or equal to the preset temperature threshold, where an output power of the vacuum generator in the second operating mode is higher than that in the first operating mode. This may mitigate the heating problem of the vacuum generator of the breast pump, and thus mitigate the suction attenuation problem of the vacuum generator caused by heating.

The above may be only the specific implementation modes of the present disclosure. It should be pointed out that for those of ordinary skill in the art, improvements and modifications may be made without departing from the principle of the present disclosure, and these improvements and modifications should also be regarded as the protection scope of the present disclosure.

## Claims

1. A control method for a breast pump, comprising:
detecting a temperature of a vacuum generator of the breast pump;
determining whether the temperature of the vacuum generator is higher than a preset temperature threshold;
controlling the vacuum generator to operate in a first operating mode in a case where the temperature of the vacuum generator is determined to be higher than the preset temperature threshold; and
controlling the vacuum generator to operate in a second operating mode in a case where the temperature of the vacuum generator is determined be lower than or equal to the preset temperature threshold; wherein an output power of the vacuum generator in the second operating mode is higher than the output power of the vacuum generator in the first operating mode.

2. The control method according to claim 1, wherein the controlling the vacuum generator to operate in the first operating mode comprises: in one operating cycle of the first operating mode, controlling the vacuum generator to operate at a first suction value in a first time period and controlling the vacuum generator to operate at a second suction value lower than the first suction value in a second time period; and
wherein the controlling the vacuum generator to operate in the second operating mode comprises: in one operating cycle of the second operating mode, controlling the vacuum generator to operate at the first suction value.

3. The control method according to claim 1, wherein the controlling the vacuum generator to operate in the first operating mode comprises: in one operating cycle of the first operating mode, controlling the vacuum generator to operate at a first suction value in a first time period and controlling the vacuum generator to stop operating in a second time period; and
wherein the controlling the vacuum generator to operate in the second operating mode comprises: in one operating cycle of the second operating mode, controlling the vacuum generator to operate at the first suction value.

4. The control method according to claim 1, wherein the controlling the vacuum generator to operate in the first operating mode in a case where the temperature of the vacuum generator is determined to be higher than the preset temperature threshold comprises:
in a case where the temperature of the vacuum generator is determined to be higher than the preset temperature threshold, further determining whether a user of the breast pump is currently in a milk letdown phase based on a milk-pumping flow rate detected in real time by the breast pump; andin a case where the user is determined to be currently in the milk letdown phase, entering the first operating mode only after the current milk letdown phase is determined to end based on the milk-pumping flow rate.

5. The control method according to claim 4, wherein the determining whether the user of the breast pump is currently in the milk letdown phase based on the milk-pumping flow rate detected in real time by the breast pump comprises:
determining whether the milk-pumping flow rate detected in real time is higher than a preset flow rate threshold; and
determining that the user is currently in the milk letdown phase in a case where the milk-pumping flow rate is higher than the preset flow rate threshold.

6. The control method according to claim 2 or 3, wherein the controlling the vacuum generator to operate in the first operating mode in a case where the temperature of the vacuum generator is determined to be higher than the preset temperature threshold comprises:
controlling a massage unit of the breast pump to work in the second time period, in a case where the temperature of the vacuum generator is determined to be higher than the preset temperature threshold.

7. The control method according to claim 2 or 3, further comprising:
setting a duration ratio of the first time period and a duration ratio of the second time period relative to the operating cycle according to an amplitude of the first suction value; wherein the greater the first suction value is, the greater the duration ratio of the second time period to the operating cycle is set to be.

8. A breast pump, comprising a processor and a memory electrically connected to the processor; wherein the memory stores a computer program, and the processor is configured to call the computer program to execute the control method according to any one of claims 1 to 7.

9. A control apparatus for a breast pump, comprising:
a detection module, configured to detect a temperature of a vacuum generator of the breast pump;
a determination module, configured to determine whether the temperature of the vacuum generator is higher than a preset temperature threshold; and
an execution module, configured to control the vacuum generator to operate in a first operating mode in a case where the temperature of the vacuum generator is determined to be higher than the preset temperature threshold, and control the vacuum generator to operate in a second operating mode in a case where the temperature of the vacuum generator is determined be lower than or equal to the preset temperature threshold; wherein an output power of the vacuum generator in the second operating mode is higher than the output power of the vacuum generator in the first operating mode.

10. A storage medium, wherein the storage medium may be configured to store a computer program, and the computer program is capable of being executed to implement the control method according to any one of claims 1 to 7.

11. A breast pump, comprising: a processor, and a temperature sensor and a vacuum generator both electrically connected to the processor;
wherein the temperature sensor is configured to detect a temperature of the vacuum generator of the breast pump;
the processor is configured to determine whether the temperature of the vacuum generator is higher than a preset temperature threshold, control the vacuum generator to operate in a first operating mode in a case where the temperature of the vacuum generator is determined to be higher than the preset temperature threshold, and control the vacuum generator to operate in a second operating mode in a case where the temperature of the vacuum generator is determined be lower than or equal to the preset temperature threshold; wherein an output power of the vacuum generator in the second operating mode is higher than the output power of the vacuum generator in the first operating mode.
